# DEMANDE DE BREVET EUROPEEN

(11) **EP 1 723 988 A1**
(43) Date de publication de la demande: **22.11.2006**
(21) Numéro de dépôt: 06300448.5
(22) Date de dépôt: 09.05.2006
(51) Int. Cl.: A61Q 1/02, A61K 8/19, A61Q 1/04, A61Q 1/08, A61Q 5/00

(54) **Composition cosmétique**

(30) Priorité: 10.05.2005 FR 0551214
(71) Demandeur: L'ORÉAL, 75008 Paris (FR)
(72) Inventeur: Dumousseaux, Christophe, Tokyo (JP)
(74) Mandataire: Tanty, François

(57) **Abrégé**

La présente invention concerne une composition cosmétique comprenant, dans un milieu physiologiquement acceptable, au moins un agent photoluminescent choisi de telle sorte que l'on observe au moins trois pics de photoluminescence de longueurs d'onde respectives différentes dans le spectre visible, lorsque la composition est éclairée sous une longueur d'onde de 365 nm.

## Description

La présente invention concerne une composition cosmétique, notamment une composition cosmétique pour le soin et/ou le maquillage de la peau, notamment du corps, des mains, du cou et du visage, des lèvres et/ou des fibres kératiniques, notamment des cils, sourcils ou cheveux.

La composition selon l'invention peut par exemple plus particulièrement viser éclaircir la peau du visage et/ou à camoufler des défauts cutanés.

Pour éclaircir la peau, il est connu d'utiliser des molécules photoluminescentes organiques, notamment des azurants optiques, en particulier celles décrites dans la demande EP 0 962 224. L'intensité de photoluminescence de certains azurants optiques est satisfaisante mais l'utilisation de telles molécules n'est pas souhaitable dans le domaine de la cosmétique en raison de leur toxicité potentielle.

De plus, les molécules photoluminescentes organiques présentent une mauvaise stabilité à la lumière et perdent ainsi leur efficacité au cours du temps après application de la composition sur la peau.

Il est par ailleurs connu d'utiliser pour éclaircir la peau, des compositions comprenant des composés inorganiques photoluminescents à base de pierres précieuses tels que décrits dans le brevet US 6 753 002 ou à base d'oxydes métalliques dopés tels que décrits dans le brevet JP 2805373. L'intensité de photoluminescence de ces composés est très faible, ce qui ne permet pas d'obtenir un effet éclaircissant satisfaisant à l'application sur la peau.

Il existe ainsi un besoin de disposer d'une composition cosmétique présentant des propriétés éclaircissantes satisfaisantes.

Il existe également un besoin pour bénéficier de compositions cosmétiques présentant de nouvelles propriétés optiques, non toxiques, et de comportement stable dans le temps, notamment photostable.

Il existe encore un besoin pour agir sur le teint de la peau, protéger des ultraviolets solaires ou créer de nouvelles couleurs de maquillage.

L'invention peut permettre de répondre à toute ou partie de ces besoins, entre autres.

Selon l'un de ses aspects, l'invention a pour objet une nouvelle composition cosmétique comprenant, dans un milieu physiologiquement acceptable, au moins un agent photoluminescent, de préférence inorganique, choisi de telle sorte que l'on observe au moins trois pics de photoluminescence de longueurs d'onde respectives différentes dans le spectre visible, lorsque la composition est éclairée sous une longueur d'onde de 365 nm.

La composition peut comprendre au moins trois agents photoluminescents différents, lesquels peuvent être associés respectivement auxdits pics, et de préférence sont inorganiques.

Selon un autre de ses aspects, l'invention a encore pour objet une composition cosmétique comprenant, dans un milieu physiologiquement acceptable, au moins trois agents photoluminescents différents.

Dans ce dernier cas, les trois agents photoluminescents peuvent être choisis de telle sorte que l'on observe au moins trois pics de photoluminescence respectifs de longueurs d'onde différentes, lorsque la composition est éclairée sous lumière ultraviolette.

La lumière ultraviolette, de longueur d'onde inférieure à 400 nm, permettant de caractériser la photoluminescence, peut être essentiellement de longueur d'onde de 365 nm, par exemple grâce à l'emploi de filtres et/ou de réseaux de diffraction, lors de l'évaluation de la photoluminescence.

La présence de plusieurs pics de photoluminescence peut permettre de créer, par synthèse additionnelle, de nouvelles couleurs et par exemple de la lumière blanche sensiblement ou toute autre lumière améliorant le teint ou renforçant la couleur d'une matière colorante additionnelle, présente dans la composition.

Les couleurs d'émission en photoluminescence peuvent être par exemple des couleurs complémentaires, telles que le rouge, le vert et le bleu par exemple.

L'invention a encore pour objet un pigment comportant :
- au moins deux, mieux trois, agents photoluminescents au moins partiellement enrobés par une matrice de préférence transparente, la taille moyenne du pigment étant de préférence inférieure à 100 nm.

La matrice peut notamment présenter un indice de réfraction inférieur ou égal à 1,8, mieux 1,6.

L'invention a encore pour objet une composition cosmétique comportant un tel pigment.

L'intervalle spectral entre deux des trois pics peut être supérieur ou égal à 40 nm environ, voire à 60 nm environ. L'intervalle spectral entre deux quelconques des trois pics peut être supérieur ou égal à 60 nm environ.

Le rapport d'intensité entre le pic de plus grande intensité et le pic de plus faible intensité peut être inférieur ou égal à 20, voire à 10, ou à 5, ou à 3. Le rapport d'intensité entre deux quelconques des trois pics peut être inférieur ou égal à 20, voire à 10, ou à 5, ou à 3. Tous les pics peuvent encore avoir sensiblement la même intensité. Un faible rapport d'intensité entre les pics peut faciliter la création de la lumière blanche par synthèse additionnelle.

L'invention a ainsi encore pour objet, selon un autre de ses aspects, une composition cosmétique comprenant, dans un milieu physiologiquement acceptable, au moins un agent photoluminescent choisi de telle sorte que l'on observe au moins trois pics de photoluminescence de longueurs d'onde respectives différentes dans le spectre visible, lorsque la composition est éclairée sous une lumière ultraviolette, le rapport entre le pic de plus grande intensité et celui de plus faible intensité étant inférieur ou égal à 20, voire à 10, ou à 5, ou à 3.

Les proportions en différents agents photoluminescents pourront être choisies de manière à avoir les intensités lumineuses recherchées. Certains agents pourront ainsi être dans une proportion plus grande si leur émissivité est plus faible.

Un pic peut avoir une longueur d'onde comprise par exemple entre 620 nm environ et 700 nm environ, ou entre 592 nm environ et 620 nm environ, ou entre 578 nm environ et 592 nm environ, ou entre 500 nm environ et 578 nm environ, ou entre 446 nm environ et 500 nm environ, ou entre 400 nm environ et 446 nm environ. Les différentes longueurs d'ondes pourront être choisies de manière à créer par synthèse additionnelle une lumière de couleur prédéfinie, par exemple une lumière blanche ou une lumière ayant une tonalité prédéfmie, par exemple correspondant à une carnation.

Le cas échéant, le choix des longueurs d'onde et/ou des proportions relatives des différents agents photoluminescents pourra le cas échéant être personnalisé en fonction de caractéristiques colorimétriques du support maquillé. La formulation de la composition pourra alors être effectuée sur un lieu de vente, par exemple, étant précédée d'une mesure de propriétés optiques de la peau de cet utilisateur. Dans une composition particulière correspondant à un exemple de mise en oeuvre de l'invention, un premier pic a une longueur d'onde comprise entre 446 nm environ et 500 nm environ, un deuxième pic a une longueur d'onde comprise entre 500 nm environ et 578 nm environ et un troisième pic a une longueur d'onde comprise entre 620 nm environ et 700 nm environ. Les agents photoluminescents émettent ainsi respectivement dans le bleu, dans le vert et dans le rouge.

Dans une autre composition donnée à titre d'exemple, un premier pic a une longueur d'onde comprise entre 400 nm environ et 446 nm environ, un deuxième pic a une longueur d'onde comprise entre 500 nm environ et 578 nm environ et un troisième pic a une longueur d'onde comprise entre 592 nm environ et 620 nm environ. Les agents photoluminescents émettent alors respectivement dans le violet, dans le vert et dans l'orange.

Dans une autre composition encore, un premier pic à une longueur d'onde comprise entre 446 nm environ et 500 nm environ, un deuxième pic a une longueur d'onde comprise entre 578 nm environ et 592 nm environ et un troisième pic a une longueur d'onde comprise entre 620 nm environ et 700 nm environ. Dans ce cas, les agents photoluminescents émettent respectivement dans le bleu, dans le jaune et dans le rouge.

### Mesure de la photoluminescence

Les mesures de photoluminescence peuvent être effectuées par exemple à l'aide du spectrofluorimètre FP6200 de la société JASCO CORPORATION, équipé pour effectuer un balayage en excitation ou en émission entre 220 nm et 730 nm. La largeur de bande en excitation et en émission est fixée à 5 nm et l'acquisition se fait par pas de 1 nm.

La composition est appliquée, lorsque pâteuse ou liquide, en film d'une épaisseur comprise entre 20 µm environ et 100 µm environ sur la partie noire d'une carte de contraste de chez Nippon Test Panel.

La composition est ensuite séchée pendant deux heures à température ambiante.

La carte de contraste est ensuite mise sur un porte-échantillon FDA-430 commercialisé par la société JASCO CORPORATION, et le spectre d'émission est mesuré en réflexion pour une longueur d'onde d'excitation de 365 nm, la largeur de la bande d'excitation et d'émission étant égale à 5 nm.

La composition, lorsqu'elle est sous forme de poudre, est d'abord introduite dans une cellule FP-1061 commercialisée par la société JASCO CORPORATION.

Cette cellule est ensuite montée sur le porte-échantillon FDA-430 commercialisé également par la société JASCO CORPORATION. Le spectre d'émission est ensuite mesuré en réflexion pour une longueur d'onde d'excitation de 365 nm.

### Agents photoluminescents

Les agents photoluminescents peuvent présenter des structures chimiques et physiques variées.

Au moins un agent photoluminescent peut être inorganique ou organique.

Au moins un agent photoluminescent peut être un pigment ou un colorant.

Au moins un agent photoluminescent peut comporter un composé métallique dopé, notamment un sel métallique dopé, le sel métallique pouvant par exemple comporter du sulfure de zinc et le dopant comporter au moins un métal. Le sel métallique peut comporter par exemple des particules de taille moyenne inférieure à 15 µm, notamment à 10 µm, par exemple inférieure à 5 µm.

Au moins un agent photoluminescent peut comporter un aluminate ou un phosphate dopé, le dopant pouvant par exemple comporter au moins une terre rare.

Au moins un agent photoluminescent peut comporter au moins un oxyde d'au moins une terre rare.

La taille moyenne des particules d'au moins un agent photoluminescent peut être inférieure ou égale à 100 nm environ, voire à 50 nm environ. Une taille moyenne des particules relativement faible peut permettre d'augmenter l'intensité de photoluminescence, et donc par exemple l'effet éclaircissant recherché.

Par « taille moyenne » des particules, on désigne la taille donnée par la distribution granulométrique statistique à la moitié de la population, dite D₅₀. Cette taille peut être mesurée par microscopie électronique, par exemple.

Au moins un agent photoluminescent peut comporter des particules enrobées au moins partiellement par une matrice, laquelle peut être transparente. Les particules peuvent être totalement encapsulées dans la matrice. La présence d'une matrice est avantageuse notamment lorsque les particules ainsi enrobées sont de taille nanométrique, notamment de taille moyenne inférieure ou égale à 100 nm, mieux à 50 nm, mieux encore à 20 nm. Cette matrice peut être polymérique ou inorganique.

La matrice peut présenter un indice de réfraction inférieur ou égal à 1,8 environ, voire inférieur ou égal à 1,6 environ.

Lorsque la matrice est polymérique, elle peut comporter par exemple du polystyrène, du polyméthylméthacrylate, du polyester, du polyéthylène ou du polyéther, notamment sous forme particulaire.

Des particules de poudre de PMMA, PE, PS ou PC encapsulant des nanoparticules de CdSe sont commercialisées par la société EVIDENT TECHNOLOGIES sous la dénomination EVI COMPOSITE™.

Lorsque la matrice est inorganique, elle peut comporter par exemple des particules de silice.

La matrice peut comporter des particules contenant au moins un, voire deux, voire trois agents photoluminescents différents.

La présence d'une matrice peut être avantageuse pour isoler le ou les agents photoluminescents du milieu environnant.

La proportion totale en agents photoluminescents dans la composition peut être comprise par exemple entre 1 % environ et 50 % environ en poids, par rapport au poids total de la composition, notamment entre 5 % environ et 20 % environ en poids, par rapport au poids total de la composition. Cette proportion pourra être choisie par exemple en fonction de la couvrance et de la saturation recherchée.

Dans un mode de mise en oeuvre de l'invention, au moins un agent photoluminescent peut comporter des particules de taille moyenne comprise entre 1 nm environ et 10 nm environ, se présentant par exemple sous forme de cristaux de CdSe, de CdSe/ZnS ou de CdTe/CdS.

Dans un autre exemple de mise en oeuvre de l'invention, au moins un agent photoluminescent peut comporter un semi-conducteur, notamment sous forme particulaire, par exemple de Si, ZnO, CdSe, ...

Le nombre d'agents photoluminescents peut être supérieur à trois.

Parmi les agents photoluminescents commercialement disponibles pouvant convenir, on peut citer entre autres l'agent photoluminescent Luminux® commercialisé par la société HONEYWELL, en particulier Luminux® Effect Blue A, Luminux® Effect Green A et Luminux® Effect Red A, ou l'agent photoluminescent Netoje® contenant une forte proportion de terres rares et commercialisé par la société NEC/TOKIN.

### Matière colorante additionnelle

La composition peut comporter au moins une matière colorante additionnelle, outre les agents photoluminescents. L'expression « matière colorante » désigne toute substance organique ou inorganique capable de procurer un effet coloré.

La matière colorante additionnelle peut être présente à raison de 0,01 à 20 % en poids, notamment de 0,05 à 10 % en poids, par exemple de 0,1 à 7 % en poids, par exemple de 0,1 à 5 % en poids, par rapport au poids total de la composition.

A titre d'exemple de matières colorantes, figurent les colorants liposolubles, les colorants hydrosolubles, les pigments et les nacres habituellement utilisés dans les compositions cosmétiques ou dermatologiques, et leurs mélanges.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les pigments peuvent être blancs ou colorés, minéraux et/ou organiques, enrobés ou non.

Les pigments peuvent être choisis parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique.

Parmi les pigments organiques, on peut citer le noir de carbone, les pigments répertoriés selon la classification D&C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les pigments peuvent avoir subi un traitement de surface.

Des pigments à effets peuvent encore être utilisés, notamment des pigments interférentiels.

### Dispositifs de conditionnement et/ou d'application

La composition peut être conditionnée sous de multiples formes, avec un applicateur ou non, selon la forme galénique.

La composition peut être conditionnée dans un dispositif de conditionnement, tel qu'un boîtier, récipient ou étui, étanche au moins avant la première utilisation. Ce dispositif de conditionnement peut être réalisé au moins partiellement avec au moins une matière thermoplastique ou en variante sans aucune matière thermoplastique. Le dispositif de conditionnement peut comporter une polyoléfine. Le dispositif de conditionnement peut également comporter au moins un élément métallique, par exemple une coupelle, une âme métallique torsadée, une charnière, une bague, un capot.

Lorsque la composition est destinée à être appliquée au moyen d'un applicateur, l'applicateur comporte par exemple une mousse, un embout floqué ou non, un feutre, une brosse, un peigne, un pinceau, un tissé, un non-tissé.

La composition peut encore imprégner un substrat, par exemple un papier, un tissé ou un non-tissé.

Lorsque présent, l'applicateur peut se loger de manière amovible sur le dispositif de conditionnement contenant la composition. En variante, l'applicateur peut être fixé à demeure sur le dispositif de conditionnement contenant la composition. Le dispositif de conditionnement peut comporter un piston ou tout autre moyen pour permettre l'alimentation de l'applicateur avec la composition.

Le dispositif de conditionnement peut comporter un organe de distribution tel qu'une pompe ou une valve, notamment lorsque la composition est liquide.

Lorsque présent, l'applicateur peut comporter une tige reliée à un organe de fermeture du dispositif de conditionnement, cet organe de fermeture pouvant également constituer un organe de préhension, le cas échéant.

Le dispositif de conditionnement contenant la composition peut être pourvu d'un fermoir ou autre moyen de fermeture, par exemple magnétique ou à encliquetage.

Le dispositif de conditionnement peut encore être pourvu d'un moyen de fermeture se fixant par vissage, friction ou encliquetage.

Le dispositif de conditionnement peut comporter des moyens d'étanchéité tels que par exemple une lèvre annulaire d'étanchéité ou un joint en élastomère, surinjecté ou rapporté.

Le dispositif de conditionnement contenant la composition peut comporter une étiquette ou une impression indiquant par exemple une marque ou un logo, cette impression ayant par exemple été réalisée par transfert à chaud ou à froid ou par sérigraphie ou par d'autres techniques d'impression.

Le dispositif de conditionnement contenant la composition peut comporter un emballage cartonné ou un blister, par exemple au moins partiellement réalisé en matière plastique transparente.

### Procédé de maquillage

L'invention a encore pour objet un procédé de maquillage et/ou de soin non thérapeutique de la peau, des lèvres et/ou des fibres kératiniques comprenant l'étape d'application sur la peau, les lèvres et/ou les fibres kératiniques d'une composition telle que définie plus haut. Préalablement, une mesure de propriétés optiques de la peau peut être effectuée, afin de formuler la composition en conséquence.

### Milieu physiologiquement acceptable

La composition selon l'invention comprend au moins un milieu physiologiquement acceptable.

Par « milieu physiologiquement acceptable », on désigne un milieu non toxique et susceptible d'être appliqué sur la peau, les lèvres ou matières kératiniques d'êtres humains. Le milieu physiologiquement acceptable est généralement adapté à la nature du support sur lequel doit être appliquée la composition ainsi qu'à l'aspect sous lequel la composition est destinée à être conditionnée.

La composition de l'invention peut dans certains exemples de mise en oeuvre comprendre une phase grasse, notamment à raison de 5 à 80 % en poids, et en particulier de 5 à 50 %, en poids par rapport au poids total de la composition.

Les huiles qui peuvent être utilisées dans la composition peuvent être choisies parmi celles classiquement utilisées dans le domaine considéré.

La composition peut comprendre une huile, notamment choisie parmi :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone et la fraction liquide du beurre de karité ;
- les esters et les éthers de synthèse, notamment d'acides gras, comme les huiles de formules R¹COOR² et R¹OR² dans laquelle R¹ représente le reste d'un acide gras ou d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le düsostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; et
- leurs mélanges.

Dans le cas où la composition se présente sous la forme d'une émulsion à phase grasse, elle peut en outre comprendre un émulsionnant, et éventuellement un coémulsionnant.

Comme émulsionnants et coémulsionnants utilisables dans l'invention, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H tels que le poly(méthylcétyl)(diméthyl)méthylsiloxane oxyéthyléné disponible sous la dénomination commerciale Abil WE09 auprès de la société DEGUSSA GOLDSCHMIDT ou le mélange de stéarate d'éthylène glycol acétyle et de tristéarate de glycéryle commercialisé par la société GUARDIAN sous la dénomination commerciale Unitwix.

L'émulsionnant et éventuellement le coémulsionnant sont généralement présents, dans la composition, en une proportion allant de 0,3 à 30 % en poids, et en particulier de 0,5 à 20 % en poids par rapport au poids total de la composition.

La composition selon l'invention peut également comprendre au moins une cire, au moins une gomme et/ou au moins un corps gras pâteux, d'origine végétale, animale, minérale ou de synthèse, siliconé ou non.

Les cires peuvent être hydrocarbonées, siliconées et/ou fluorées, composées éventuellement des fonctions ester ou hydroxyle. Elles peuvent être notamment d'origine naturelle.

La cire peut représenter de 0,01 à 10 % en poids, notamment de 0,1 à 5 % en poids, par rapport au poids total de la composition. Selon un mode de réalisation, la composition peut être exempte de cire.

La composition peut également comprendre au moins une charge minérale ou organique, enrobée ou non, notamment à titre d'agent matifiant, par exemple l'oxyde de zinc et de zircone, la silice, l'alumine, le nitrure de bore, le talc, la séricite, le mica, les argiles, l'amidon et ses dérivés, notamment l'amidon réticulé par l'anhydride octénylsuccinique commercialisé par la société NATIONAL STARCH sous la dénomination DRY FLO PLUS (28-1160), les dispersions aqueuses de styrène acrylique, les particules de résine de mélamine-formaldéhyde ou d'urée-formaldéhyde, les dispersions aqueuses de polytétrafluoroéthylène, les microdispersions de cires, les copolymères vinylpyrrolidone/1-triacontène, les polymères hydrodispersibles contenant des unités à LCST, les cires et résines de silicone, notamment les microbilles de résine de silicone telles que celles commercialisées sous la dénomination TOSPEARL par la société TOSHIBA SILICONE, les poudres expansées telles que les microsphères creuses et notamment les microsphères commercialisées sous la dénomination EXPANCEL par la société KEMANORD PLAST ou sous la dénomination MICROPEARL F 80 ED par la société MATSUMOTO, les microsphères de terpolymère expansé de chlorure de vinylidène, d'acrylonitrile et de méthacrylate, les particules de polyamide, par exemple de Nylon® ou celles vendues sous la dénomination ORGASOL par la société ATOCHEM, les microbilles de cellulose, les fibres, les poudres de polyéthylène, les microsphères à base de copolymères acryliques, telles que celles en copolymère diméthacrylate d'éthylène glycol/méthacrylate de lauryle vendues par la société DOW CORNING sous la dénomination de POLYTRAP et leurs mélanges.

La charge, notamment lorsqu'utilisée comme agent matifiant, peut être présente en une teneur allant de 0,1 à 80 % en poids par rapport au poids total de la composition, par exemple une teneur comprise entre 0,1 % et 10 % en poids par rapport au poids total de la composition.

Il est bien entendu que le choix de cet agent matifiant, de même que celui de la quantité utilisée, seront effectués par l'homme du métier de manière à ne pas nuire aux propriétés recherchées.

La composition peut contenir également au moins un adjuvant habituel dans le domaine cosmétique, tel que les charges, par exemple choisies dans la liste précitée, les gélifiants hydrophiles ou lipophiles, les actifs, hydrosolubles ou liposolubles, les conservateurs, les hydratants, tels que les polyols et notamment la glycérine, les séquestrants, les antioxydants, les solvants, les parfums, les filtres solaires physiques et chimiques, notamment aux UVA et/ou aux UVB, les absorbeurs d'odeur, les ajusteurs de pH (acides ou bases) et leurs mélanges.

Les quantités de ces différents adjuvants sont celles classiquement utilisées dans le domaine considéré, par exemple de 0,01 à 20 % du poids total de la composition.

En tout état de cause, ces adjuvants, ainsi que leurs proportions, seront choisis de manière à ne pas nuire aux propriétés recherchées selon l'invention.

A titre d'actifs, on peut citer en particulier :
- les actifs connus pour leur activité sur le vieillissement de la peau comme les agents keratolytiques ou prodesquamants, par exemple les α-hydroxy-acides, les β-hydroxy-acides, les α-céto-acides, les β-céto-acides, les rétinoïdes et leurs esters, le rétinal, l'acide rétinoïque et ses dérivés ;
- les vitamines, telles que par exemple les vitamines A, B3, PP, B5, E, K1 et/ou C, et les dérivés de ces vitamines et notamment leurs esters ;
- les agents anti-radicaux libres ;
- les filtres solaires ;
- les agents hydratants comme les polyols ;
- les céramides ;
- la DHEA et ses dérivés ;
- le coenzyme Q10 ;
- les agents blanchissants et dépigmentants par action biologique complémentaires comme l'acide kojique, les extraits de scutellaire, de mûrier, de réglisse et/ou de camomille, les dérivés de para-aminophénols, l'arbutine et leurs dérivés, et leurs mélanges ;
- les actifs utiles pour les peaux grasses ou mixtes, tels que les sels de zinc et en particulier l'oxyde de zinc et le gluconate de zinc ;
- les antibactériens comme l'acide salicylique et ses dérivés tels que l'acide n-octanoyl-5-salicylique, le triclosan, le lipacide, la capryloylglycine, l'extrait de clou de girofle, l'octopirox, l'hexamidine, l'acide azélaïque et ses dérivés ;
- les actifs anti-acné, ou encore,
- les extraits de plantes veinotoniques tels que les extraits de petit houx et/ou de marron d'Inde ; les bases xanthiques telles que la caféine.

La composition selon l'invention peut se présenter sous toutes les formes galéniques normalement utilisées dans le domaine cosmétique, normalement utilisées pour une application topique : émulsions directes, inverses ou multiples, gels, crèmes, solutions, suspensions, lotions, poudres libres, compactes et sticks.

Elle peut plus précisément se présenter sous forme de solution huileuse éventuellement gélifiée, d'émulsion de consistance liquide ou semi-liquide du type lait, obtenue par dispersion d'une phase grasse dans une phase aqueuse (H/E) ou inversement (E/H), d'une émulsion triple (E/H/E ou H/E/H), ou de suspension ou émulsion de consistance molle, semi-solide ou solide de type crème ou gel, ou encore de microémulsions, de microcapsules, de microparticules ou d'une dispersion vésiculaire de type ionique (liposomes ou oléosomes) et/ou non ionique (niosomes) et/ou d'une dispersion de nanocapsules ou nanosphères.

La composition cosmétique selon l'invention peut notamment se présenter sous forme de composition de soin, de maquillage et/ou de protection solaire.

Plus particulièrement, la composition selon l'invention se présente sous la forme d'un produit de maquillage du visage, notamment de la peau et/ou des lèvres, par exemple sous forme de fond de teint.

La composition selon l'invention peut encore se présenter sous la forme d'une composition de soin et/ou de maquillage de la peau, des lèvres et/ou des fibres kératiniques.

La composition peut par exemple se présenter sous forme de gel anti-cernes, de crème de soin ou de lotion photoprotectrice, notamment des UV.

### Exemples

Les exemples figurant ci-après sont présentés à titre illustratif et non limitatif de l'invention. Les pourcentages sont exprimés en poids par rapport au poids total de la composition.

### Exemple 1 : Fond de teint

| | % |
|---|---|
| Talc | 37.8 |
| Séricite | 31.5 |
| Mica | 4.5 |
| Dioxyde de Titane | 4.9 |
| Oxydes de fer | 2.0 |
| Stéarate de zinc | 0.9 |
| Paraffine liquide | 3.6 |
| Phényl Triméthicone | 4.5 |
| Parabens | 0.3 |
| Lumilux Effect Blue A* | 3 |
| Lumilux Effect Green A** | 2 |
| Lumilux Effect Red A*** | 5 |

L'agent photoluminescent Lumilux® est commercialisé par la société HONEYWELL.
* L'agent photoluminescent Lumilux Effect Blue A comporte du sulfure de zinc dopé à l'argent (ZnS:Ag) sous forme de particules présentant une taille moyenne de 20 µm. Il émet dans le bleu avec un pic à 450 nm et une bande large d'émission entre 400 et 520 nm sous lumière UV.
**L'agent photoluminescent Lumilux Effect Green A comporte du sulfure de zinc dopé au cuivre (ZnS:Cu) sous forme de particules présentant une taille moyenne de 8 µm. Il émet dans le vert avec un pic à 530 nm et une bande large d'émission entre 420 et 660 nm sous lumière UV.
***L'agent photoluminescent Lumilux Effect Red A comporte du sulfure d'yttrium dopé à l'europium (Y₂O₂S:Eu) sous forme de particules présentant une taille moyenne de 10 µm. Il émet dans le rouge avec un maxima à 620 nm et a une bande d'émission relativement étroite sous lumière UV.

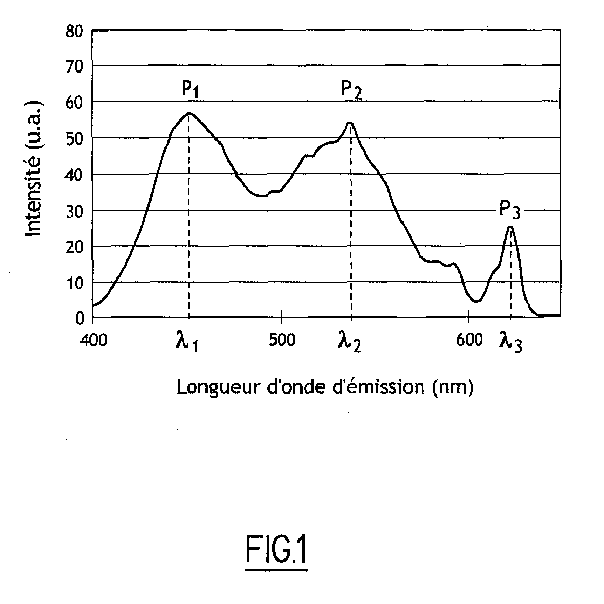

On a représenté de manière schématique sur la figure 1, le spectre de photoluminescence de la composition de l'exemple 1 en fonction de la longueur d'onde.

Dans cet exemple, on peut voir que le pic P1 a une longueur d'onde λ₁ de 450 nm environ, l'agent photoluminescent correspondant ayant une bande d'émission comprise entre 400 et 520 nm environ. Le pic P2 a une longueur d'onde λ₂ de 530 nm environ, l'agent photoluminescent correspondant ayant une bande d'émission comprise entre 420 et 660 nm. Enfin, le pic P3 a une longueur d'onde λ₃ de 620 nm, l'agent photoluminescent ayant une bande d'émission comprise entre 600 et 635 nm.

### Exemple 2 : Fond de teint liquide

| | % |
|---|---|
| Phase I | |
| Cétyl diméthicone copolyol/ | |
| polyglycéryl-4 Isostearate/ laurate d'hexyl | 8 |
| Diméthicone | 4.8 |
| Cyclométhicone | 5.2 |
| Isododécane | 2.8 |
| Neopentanoate d'isostéaryl | 0.8 |
| Gel de Bentone | 8 |
| Oxyde de titane | 5 |
| Oxyde de fer | 1 |
| Phase II | 43.2 |
| Eau | 5.6 |
| Butylène glycol | 0.8 |
| Sulfate de Magnésium | 0.8 |
| Conservateurs | |
| Phase III | 4 |
| Talc | 3 |
| Netoje A-B¹ | 2 |
| Netoje S-G² | 5 |
| Netoje A-R³ | |

Les particules Netoje® sont commercialisées par la société NEC/TOKIN.

¹ L'agent photoluminescent Netoje A-B émet dans le bleu sous excitation de 365 nm.

² L'agent photoluminescent Netoje S-G émet dans le vert sous excitation de 365 nm.

³ L'agent photoluminescent Netoje A-R émet dans le rouge sous excitation de 365 nm.

### Mode opératoire

Les phases I et II sont mélangées séparément, la phase II est ensuite rajoutée à la phase I à l'aide d'une turbine adéquate pour réaliser l'émulsification. La phase III est ensuite rajoutée à l'émulsion.

L'expression « comportant un « doit être comprise comme étant synonyme de l'expression « comportant au moins un », sauf si le contraire est spécifié. Les intervalles doivent être compris bornes incluses sauf si le contraire est spécifié.

## Revendications

1. Composition cosmétique comprenant, dans un milieu physiologiquement acceptable, au moins un agent photoluminescent choisi de telle sorte que l'on observe au moins trois pics de photoluminescence de longueurs d'onde respectives différentes dans le spectre visible, lorsque la composition est éclairée sous une longueur d'onde de 365 nm.

2. Composition cosmétique comprenant, dans un milieu physiologiquement acceptable, au moins trois agents photoluminescents différents.

3. Composition selon la revendication 1, **caractérisée par le fait qu'**elle comprend au moins trois agents photoluminescents différents.

4. Composition cosmétique selon la revendication 2, **caractérisée par le fait que** les trois agents photoluminescents sont choisis de telle sorte que l'on observe au moins trois pics de photoluminescence respectivement associés à ces agents photoluminescents, de longueurs d'onde respectives différentes lorsque la composition est éclairée sous lumière ultraviolette.

5. Composition cosmétique comprenant, dans un milieu physiologiquement acceptable, au moins un agent photoluminescent choisi de telle sorte que l'on observe au moins trois pics de photoluminescence de longueurs d'onde respectives différentes dans le spectre visible, lorsque la composition est éclairée sous une longueur d'onde de 365 nm, le rapport d'intensité entre un pic de plus grande intensité et un pic de plus faible intensité étant inférieur ou égal à 20.

6. Composition selon la revendication 5, dans laquelle le rapport d'intensité entre le pic de plus grande intensité et le pic de plus faible intensité est inférieur ou égal à 10.

7. Composition selon la revendication 5, dans laquelle le rapport d'intensité entre le pic de plus grande intensité et le pic de plus faible intensité est inférieur ou égal à 5.

8. Composition selon la revendication 5, dans laquelle le rapport d'intensité entre le pic de plus grande intensité et le pic de plus faible intensité est inférieur ou égal à 3.

9. Composition selon l'une quelconque des revendications 1 à 8, **caractérisée par le fait que** l'intervalle spectral entre au moins deux des trois pics est supérieur ou égal à 40 nm environ.

10. Composition selon l'une quelconque des revendications 1 à 8, **caractérisée par le fait que** l'intervalle spectral entre deux quelconques des trois pics est supérieur ou égal à 40 nm environ.

11. Composition selon l'une quelconque des revendications 1 à 8, **caractérisée par le fait que** l'intervalle spectral entre au moins deux des trois pics est supérieur ou égal à 60 nm environ.

12. Composition selon l'une quelconque des revendications 1 à 8, **caractérisée par le fait que** l'intervalle spectral entre deux quelconques des trois pics est supérieur ou égal à 60 nm environ.

13. Composition selon l'une quelconque des revendications précédentes, **caractérisée par le fait qu'**au moins un agent photoluminescent est inorganique.

14. Composition selon la revendication 1, **caractérisée par le fait que** le rapport d'intensité entre un pic de plus grande intensité et un pic de plus faible intensité est inférieur ou égal à 20, mieux à 10, ou à 5, ou à 3.

15. Composition selon la revendication 1, **caractérisée par le fait que** le rapport d'intensité entre deux quelconques des trois pics est inférieur ou égal à 20, mieux à 10, ou à 5, ou à 3.

16. Composition selon l'une quelconque des revendications précédentes, **caractérisée par le fait qu'**au moins un agent photoluminescent comporte un pigment.

17. Composition selon l'une quelconque des revendications précédentes, **caractérisée par le fait qu'**au moins un agent photoluminescent comporte un colorant.

18. Composition selon l'une quelconque des revendications précédentes, **caractérisée par le fait qu'**au moins un agent photoluminescent comporte un sel métallique dopé par au moins un dopant.

19. Composition selon la revendication précédente, **caractérisée par le fait que** le sel métallique comporte du sulfure de zinc.

20. Composition selon la revendication 18, **caractérisée par le fait que** le dopant comporte au moins un métal.

21. Composition selon la revendication 18, **caractérisée par le fait que** le sel métallique comporte des particules de taille moyenne inférieure à 15 µm, notamment à 10 µm ou à 5 µm.

22. Composition selon l'une quelconque des revendications précédentes, **caractérisée par le fait qu'**au moins un agent photoluminescent comporte un aluminate ou un phosphate dopé par au moins un dopant.

23. Composition selon la revendication précédente, **caractérisée par le fait que** le dopant comporte au moins une terre rare.

24. Composition selon l'une quelconque des revendications précédentes, **caractérisée par le fait qu'**au moins un agent photoluminescent comporte au moins un oxyde d'au moins une terre rare.

25. Composition selon l'une quelconque des revendications précédentes, **caractérisée par le fait qu'**au moins un agent photoluminescent est particulaire et **par le fait que** la taille moyenne des particules de cet agent photoluminescent est inférieure ou égale à 100 nm environ.

26. Composition selon la revendication précédente, **caractérisée par le fait que** ladite taille moyenne est inférieure ou égale à 50 nm environ, notamment comprise entre 1 nm et 10 nm environ.

27. Composition selon l'une quelconque des revendications précédentes, **caractérisée par le fait qu'**au moins un agent photoluminescent comporte des particules enrobées au moins partiellement dans une matrice.

28. Composition selon la revendication 27, **caractérisée par le fait que** les particules sont totalement encapsulées dans la matrice.

29. Composition selon l'une des revendications 27 et 28, **caractérisée par le fait que** la matrice est polymérique.

30. Composition selon l'une des revendications 27 et 28, **caractérisée par le fait que** la matrice est inorganique.

31. Composition selon l'une quelconque des revendications 27 à 30, **caractérisée par le fait que** la matrice présente un indice de réfraction inférieur ou égal à 1,8 environ.

32. Composition selon l'une quelconque des revendications 27 à 30, **caractérisée par le fait que** la matrice présente un indice de réfraction inférieur ou égal à 1,6 environ.

33. Composition selon la revendication 29, **caractérisée par le fait que** la matrice comporte du polystyrène, du polyméthylméthacrylate, du polyester, du polyéthylène ou du polyéther, notamment sous forme particulaire.

34. Composition selon la revendication 30, **caractérisée par le fait que** la matrice comporte de la silice, notamment des particules de silice.

35. Composition selon l'une quelconque des revendications 27 à 33, **caractérisée par le fait que** la matrice contient des particules contenant au moins deux, voire au moins trois, agents photoluminescents différents.

36. Composition selon l'une quelconque des revendications précédentes, **caractérisée par le fait que** la proportion en agents photoluminescents dans la composition est comprise entre 1 % environ et 50 % environ en poids, par rapport au poids total de la composition.

37. Composition selon l'une quelconque des revendications précédentes, **caractérisée par le fait que** la proportion en agents photoluminescents est comprise entre 5 % environ et 20 % environ en poids, par rapport au poids total de la composition.

38. Composition selon l'une quelconque des revendications précédentes, **caractérisée par le fait qu'**au moins un agent photoluminescent comporte des particules de taille moyenne comprise entre 1 nm environ et 10 nm environ.

39. Composition selon l'une quelconque des revendications précédentes, **caractérisée par le fait qu'**au moins un agent photoluminescent comporte des cristaux de CdSe, de CdSe/ZnS ou de CdTe/CdS.

40. Composition selon l'une quelconque des revendications précédentes, **caractérisée par le fait qu'**au moins un agent photoluminescent comporte au moins un semi-conducteur, notamment choisi parmi Si, ZnO, CdSe.

41. Composition selon l'une quelconque des revendications précédentes, **caractérisée par le fait que** les agents photoluminescents sont choisis de telle sorte que la lumière résultante émise par les agents photoluminescents soit une lumière sensiblement blanche.

42. Composition selon l'une quelconque des revendications précédentes, **caractérisée par le fait que** le nombre d'agents photoluminescents est supérieur à trois.

43. Composition selon l'une quelconque des revendications précédentes, **caractérisée par le fait qu'**un pic a une longueur d'onde comprise entre 620 nm environ et 700 nm environ.

44. Composition selon l'une quelconque des revendications précédentes, **caractérisée par le fait qu'**un pic a une longueur d'onde comprise entre 592 nm environ et 620 nm environ.

45. Composition selon l'une quelconque des revendications précédentes, **caractérisée par le fait qu'**un pic a une longueur d'onde comprise entre 578 nm environ et 592 nm environ.

46. Composition selon l'une quelconque des revendications précédentes, **caractérisée par le fait qu'**un pic a une longueur d'onde comprise entre 500 nm environ et 578 nm environ.

47. Composition selon l'une quelconque des revendications précédentes, **caractérisée par le fait qu'**un pic a une longueur d'onde comprise entre 446 nm environ et 500 nm environ.

48. Composition selon l'une quelconque des revendications précédentes, **caractérisée par le fait qu'**un pic a une longueur d'onde comprise entre 400 nm environ et 446 nm environ.

49. Composition selon l'une quelconque des revendications précédentes, **caractérisée par le fait qu'**un premier pic a une longueur d'onde comprise entre 446 nm environ et 500 nm environ, un deuxième pic a une longueur d'onde comprise entre 500 nm environ et 578 nm environ et un troisième pic a une longueur d'onde comprise entre 620 nm environ et 700 nm environ.

50. Composition selon l'une quelconque des revendications précédentes, **caractérisée par le fait qu'**un premier pic a une longueur d'onde comprise entre 400 nm environ et 446 nm environ, un deuxième pic a une longueur d'onde comprise entre 500 nm environ et 578 nm environ et un troisième pic a une longueur d'onde comprise entre 592 nm environ et 620 nm environ.

51. Composition selon l'une quelconque des revendications précédentes, **caractérisée par le fait qu'**un premier pic a une longueur d'onde comprise entre 446 nm environ et 500 nm environ, un deuxième pic a une longueur d'onde comprise entre 578 nm environ et 592 nm environ et un troisième pic a une longueur d'onde comprise entre 620 nm environ et 700 nm environ.

52. Composition selon l'une quelconque des revendications précédentes, **caractérisée par le fait qu'**elle comporte au moins une matière colorante additionnelle, outre les agents photoluminescents.

53. Composition selon la revendication 52, **caractérisée par le fait que** la matière colorante additionnelle est choisie dans le groupe constitué par les colorants liposolubles, les colorants hydrosolubles, les pigments et les nacres habituellement utilisés dans les compositions cosmétiques ou dermatologiques, et leurs mélanges.

54. Dispositif de conditionnement contenant une composition telle que définie dans l'une quelconque des revendications précédentes, et éventuellement un applicateur pour appliquer la composition.

55. Procédé de maquillage et/ou de soin non thérapeutique de la peau, des lèvres et/ou des fibres kératiniques comprenant l'étape d'application sur la peau, les lèvres et/ou les fibres kératiniques d'une composition telle que définie selon l'une quelconque des revendications 1 à 53.
